**Europäisches Patentamt**

**European Patent Office**

**Office européen des brevets**

(19)

(11) Veröffentlichungsnummer: **0 125 704**
**B1**

(12)

# EUROPÄISCHE PATENTSCHRIFT

(45) Veröffentlichungstag der Patentschrift:
14.01.87

(51) Int. Cl.⁴: **A 61 M 5/30**

(21) Anmeldenummer: **84106147.6**

(22) Anmeldetag: **09.04.82**

(54) Kolbenpumpe für nadellose Injektionsgeräte.

(30) Priorität: **16.04.81 DE 3115372**

(43) Veröffentlichungstag der Anmeldung:
**21.11.84 Patentblatt 84/47**

(45) Bekanntmachung des Hinweises auf die Patenterteilung:
**14.01.87 Patentblatt 87/3**

(84) Benannte Vertragsstaaten:
**CH DE FR GB LI**

(56) Entgegenhaltungen:
**US-A-3 908 651**

(73) Patentinhaber: **HOECHST AKTIENGESELLSCHAFT,
Postfach 80 03 20, D-6230 Frankfurt am Main 80
(DE)**

(72) Erfinder: **Dettbarn, Hans-Jürgen, Rehbocks Ecke
4, D-3550 Marburg (DE)**
Erfinder: **Zimmermann, Jozef, Auf der Krautweide
11, D-6231 Sulzbach (DE)**

EP 0 125 704 B1

LIBER, STOCKHOLM 1987

## Beschreibung

Die Erfindung betrifft eine Kolbenpumpe für nadellose Injektionsgeräte, bei der das Pumpengehäuse eine Halterung für einen Behälter für das zu injizierende Medium aufweist, die aus einem mit Hohlnadel zur Aufnahme des Behälters versehenen Stutzen, der Teil des Pumpengehäuses ist, und einem Verbindungselement bestehend aus einem Nadelstutzen, der die Hohlnadel trägt, zum Verbinden des Behälters mit dem Stutzen besteht, und das Pumpengehäuse einen Einlaßkanal aufweist, der mit einem Kanal im Stutzen in Verbindung steht.

Kolbenpumpen der genannten Art sind aus der deutschen Auslegeschrift 14 91 833 und den US-Patentschriften 3 526 225 und 3 908 651 bekannt.

Nach der deutschen Auslegeschrift 14 91 833 ist am Gehäuse der Impfstoffpumpe eine Lagerplatte befestigt, die ein Medikamenten- und ein Luftröhrchen sowie den Medikamentenbehälter trägt. Festgeklemmt wird der Medikamentenbehälter durch ein aufspannbares Widerlager - eine Art Bügel-, das aus einem Stopfen, einer Konsole und teleskopartig angeordneten Rohren besteht, die an der Lagerplatte befestigt sind. Diese Konstruktion ist umständlich in der Handhabung. Behälter mit unterschiedlichem Durchmesser sind nur begrenzt verwendbar. Ferner erhöht diese Art der Halterung für den Behälter das Gewicht der Kolbenpumpe und damit das des Injektionsgerätes erheblich.

Nach der US-Patentschrift 3 526 225 wird der Behälter für den Impfstoff durch eine federnde Halterung aus Draht auf dem Nadelstutzen gehalten. Nachteilig bei dieser Art der Hal-Drahtbügel gesichert wird, jedoch nicht gegen Herausrutschen. Auch der Nadelstutzen weist keinerlei Elemente auf, die ein Auseinandergleiten der Verbindung Behälter Nadelstutzen verhindern würde. Ferner sind auch hier Behälter mit unterschiedlichem Durchmesser nur begrenzt verwendbar.

Nach der US Patentschrift 3 908 651 ist ein Injektionsgerät mit einer Haltevorrichtung für den Impfstoffbehälter bekannt. Diese besteht aus einem Befestigungsteil mit Befestigungsring sowie einem Nadelhalter. Die Vorrichtung ist nur für Behälter mit speziellem Durchmesser geeignet. Der Nadelhalter weist keine Elemente auf, die ein Auseinandergleiten der Verbindung Behälter Nadelhalter verhindern würde.

Hier will die Erfindung Abhilfe schaffen. Die Erfindung wie sie in den Ansprüchen gekennzeichnet ist, löst die Aufgabe dadurch, daß der Nadelstutzen der Halterung der eingangs gennanten kolbenpumpe einen Dorn mit einer Hinterschneidung aufweist, durch den die Hohlnadel geführt ist, und daß um den . Nadelstutzen herum teleskopartig ein Federelement angeordnet ist, das den Behälter gegen die Hinterschneidung preßt. In der Hohlnadel kann ein Belüftungsröhrchen angeordnet sein. Die Nadel kann durch den Nadelstutzen hindurch in den Stutzen ragen, der zur Abdichtung der Nadel mit einem 0-Ring versehen ist.

Der durch die Erfindung erreichte Vorteil ist im wesentlichen darin zu sehen, daß der Behälter für das zu injizierende Medium auf einfache Weise starr mit der Kolbenpumpe verbunden werden kann. Diese Art der Verbindung ist für alle handelsüblichen Behälter mit der erfindungsgemäßen Halterung möglich.

Im folgenden wird die Erfindung anhand von lediglich einen Ausführungsweg darstellenden Zeichnung näher erläutert: Es zeigt

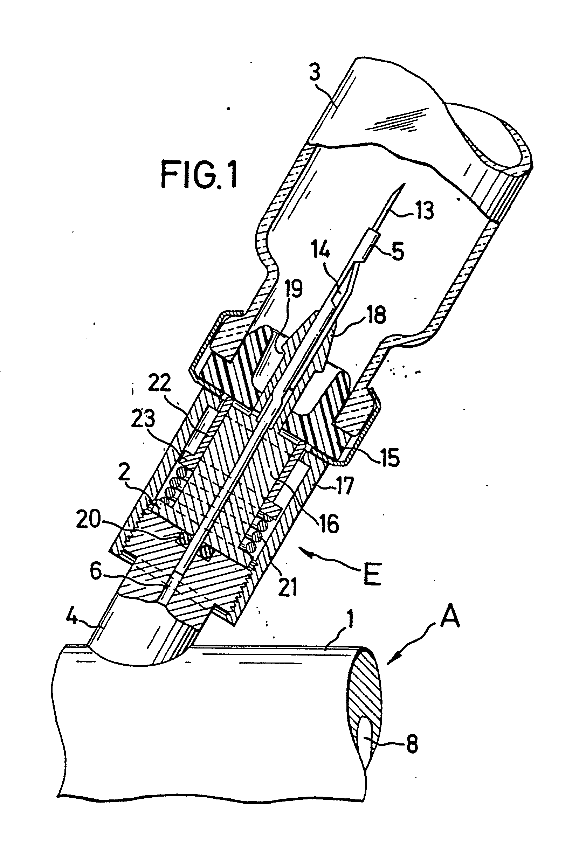

Figur 1 eine Seitenansicht der Kolbenpumpe mit aufgestecktem Behälter und gespanntem Federelement teilweise geschnitten;

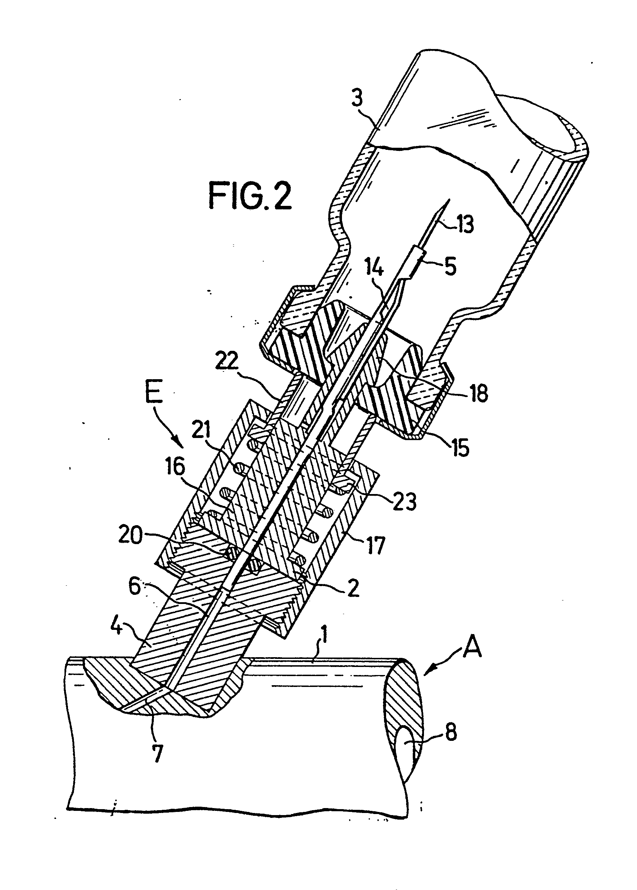

Figur 2 den aufgesteckten Behälter gegen die Hinterschneidung gepreßt.

Das Pumpengehäuse (1) der Kolbenpumpe A für nadellose Injektionsgeräte trägt eine Halterung E für einen Behälter (3), in dem sich das zu injizierende Medium befindet. Die Halterung E besteht aus dem Stutzen (4) und einem Verbindungselement, das den Behälter (3) mit dem Stutzen (4) verbindet. Der Stutzen (4), der Teil des Pumpengehäuses ist, dann eine Hohlnadel (5) tragen. Das zu injizierende Medium gelangt über die Hohlnadel (5), den Kanal (6) im Stutzen (4) und der Einlaßkanal (7) im Pumpengehäuse (1) vom Behälter (3) in die Pumpenkammer (8).

Das Verbindungselement besteht aus einem Nadelstutzen (16), der zum Beispiel mittels einer Uberwurfmutter (17) mit dem Stutzen (4) verbunden ist. Der Nadelstutzen (16) weist einen Dorn (18) mit Hinterschneidungen (19) auf, durch den die Hohlnadel (5) geführt ist. Es kann zweckmäßig sein, daß die Hohlnadel (5) in den Stutzen (4) hineinragt. Sie wird dann dort mittels 0-Ring (20) abgedichtet. Um den Nadelstutzen herum ist ein Federelement teleskopartig angeordnet, Es besteht aus einer Druckfeder (21) und einer Hülse (22) mit Bördel (23). Die Druckfeder (21) stützt sich einerseits auf dem Bördel (23) und andererseits auf den Flansch (2) des Nadelstutzens (16) ab. Beim Aufsetzen des Behälters (3) auf den Nadelstutzen (16) wird de Verschlußstopfen (15) des Behälters (3) vom Belüftungsröhrchen (13), der Hohlnadel (5) und dem Dorn (18) durchdrungen. Der Behälter (3) wird soweit über den Dorn (18) geschoben, bis der Verschlußstopfen (15) hinter die-Hinterschneidung (19) des Dornes (18) greift. Hierbei wird die Druckfeder (21) über die Hülse (22) zusammengedrückt, Beim Loslassen des Behälters (3) schiebt die Druckfeder (21) den Behälter (3) über die Hülse (22) soweit zurück, bis der Verschlußstopfen (15) an der Hinterschneidung (19) des Dornes (18) anliegt.

In der Hohlnadel (5) ist ein Belüftungsröhrchen (13) angeordnet. Dieses wird durch eine seitliche Offnung (I4) in die Hohlnadel (5) eingeführt. Durch die seitliche Offnung (IV) der Hohlnadel (5) gelangt das zu injizierende Medium in die Hohlnadel.

**Patentansprüche**

1. Kolbenpumpe (A) für nadellose Injektionsgeräte, bei der das Pumpengehäuse (1) eine Halterung (E) für einen Behälter (3) für das zu injizierende Medium aufweist, die aus einem mit Hohlnadel (5) zum Aufnehmen des Behälters (3) versehenen Stutzen (4), der Teil des Pumpengehäuses (1) ist, und einem Verbindungselement bestehend aus einem Nadelstutzen (16), der die Hohlnadel (5) trägt zum Verbinden des Behälters (3) mit dem Stutzen (4) besteht, und das Pumpengehäuse (1) einen Einlaßkanal (7) aufweist, der mit einem Kanal (6) im Stutzen (4) in Verbindung steht, dadurch gekennzeichnet, daß der Nadelstutzen (16) einen Dorn (18) mit einer Hinterschneidung (19) aufweist, durch den die Hohlnadel (5) geführt ist, und daß um den Nadelstutzen (16) herum teleskopartig ein Federelement (21, 22, 23) angeordnet ist, das den Behälter (3) gegen die Hinterschneidung (19) preßt.

2. Kolbenpumpe nach Anspruch 1, dadurch gekennzeichnet. daß in der Hohlnadel (5) ein Belüftungsröhrchen (13) angeordnet ist.

3. Kolbenpumpe nach Anspruch 1, dadurch gekennzeichnet, daß die Hohlnadel (5) durch den Nadelstutzen (18) hindurch in den Stutzen (4) ragt, der zur Abdichtung der Hohlnadel mit einem O-Ring (20) versehen ist.

**Claims**

1. A piston pump (A) for needle-less injection instruments, in which the pump housing (1) has a mounting (E) for a vessel (3) for the medium to be injected, which consists of a pipe end (4) which is provided with a hollow needle (5) for receiving the vessel (3) and which is part of the pump housing (1) and of a connecting element which consists of a needle connection (16) carrying the hollow needle (5) for connecting the vessel (3) to the pipe end (4), the pump housing (1) having an inlet channel (7) connected to a channel (6) in the pipe end (4), wherein the needle connection (16) has a peg (16) with an undercut (19), through which the hollow needle (5) is guided, and that there being arranged telescopically round the needle connection (16) a spring element (21, 22, 23) which presses the vessel (3)against the undercut (19).

2. The piston pump as claimed in claim 1, wherein a venting tube (13) is located in the hollow needle (5).

3. The piston pump as claimed in claim 1, wherein the hollow needle (5) projects through the needle connection (16) into the pipe end (4) which is provided with an O-ring (20) for sealing off the hollow needle.

**Revendications**

1 - Pompe à piston (A) pour appareils d'injection sans aiguille, dans laquelle le corps (1) de la pompe présente une fixation (E) destinée à recevoir le réservoir (3) contenant le milieu à injecter et qui est composée, d'une part, d'une tubulure (4) munie d'une aiguille creuse (5) et destinée à recevoir le réservoir (3), tubulure qui fait partie du corps (1) de la pompe, et, d'autre part, d'un élément de liaison composé lui-même d'une tubulure d'aiguille (16) qui porte l'aiguille creuse (5), cet élément servant à relier le réservoir (3) à la tubulure (4), et où le corps (1) de la pompe présente un canal d'entrée (7) qui communique avec un canal (6) ménagé dans la tubulure (4), caractérisée en ce que la tubulure d'aiguille (16) présente un mandrin (18) muni d'une contre-dépouille (19) et à travers lequel passe l'aiguille creuse (5) et en ce qu'autour de la tubulure d'aiguille (16), est disposé télescopiquement un élément élastique (21, 22, 23) qui presse le réservoir (3) contre la contre-dépouille (19).

2 - Pompe à piston selon la revendication 1, caractérisée en ce qu'un petit tube d'entrée d'air (13) est agencé dans l'aiguille creuse (5).

3 - Pompe à piston selon la revendication 1, caractérisée en ce que l'aiguille creuse (5) pénètre dans la tubulure (4) en traversant la tubulure d'aiguille (16), la tubulure (4) étant munie d'une bague torique (20) pour fermer le joint sur l'aiguille creuse.

FIG.1

FIG.2